# EUROPEAN PATENT APPLICATION

(11) **EP 3 058 956 A1**
(43) Date of publication of application: **24.08.2016**
(21) Application number: 15000507.2
(22) Date of filing: 23.02.2015
(51) Int. Cl.: A61K 41/00, A61N 5/10, A61K 47/48

(54) **Combined vaccination/radioterapy for cancer treatment**

(71) Applicant: Institut Curie, 75005 Paris (FR); Centre National de la Recherche Scientifique (CNRS), 75794 Paris Cedex 16 (FR); Institut Gustave Roussy (IGR), 94805 Villejuif Cedex (FR); Assistance Publique Hôpitaux de Paris, 75004 Paris (FR); Institut National de la Santé et de la Recherche Médicale (I.N.S.E.R.M.), 75654 Paris Cedex 13 (FR); Université René Descartes (Paris V), 75006 Paris (FR)
(72) Inventor: Deutsch, Eric, 75004 Paris (FR); Johannes, Ludger, 92400 Courbevoie (FR); Mondini, Michèle, 75013 Paris (FR); Nizard, MMevyn, 94260 Fresnes (FR); Perfettini, Jean-Luc, 77100 Meaux (FR); Tartour, Erice, 75003 Paris (FR); Tran, Thi, 75018 Paris (FR)
(74) Representative: Barbot, Willy

(57) **Abstract**

The present invention concerns a a composition for use in the prevention or treatment of a subject suffering from a cancer in combination with radiotherapy, wherein said composition comprises a conjugate, eventually associated with pharmaceutically acceptable carrier, said conjugate comprising i) the Shiga Toxin B subunit (STxB), a fragment or a derivative thereof; and ii) at least one epitope associated with said cancer.

SEQUENCE LISTING

<110>  Institut Gustave Roussy
       Institut Curie
       INSERM

<120>  COMBINED VACCINATION/RADIOTERAPY FOR CANCER TREATMENT

<130>  STX-B-0001-EP1

<160>  5

<170>  PatentIn version 3.5

<210>  1
<211>  89
<212>  PRT
<213>  Shigella dysenteriae

<400>  1

Met Lys Lys Thr Leu Leu Ile Ala Ala Ser Leu Ser Phe Phe Ser Ala
1               5                   10                  15

Ser Ala Leu Ala Thr Pro Asp Cys Val Thr Gly Lys Val Glu Tyr Thr
            20                  25                  30

Lys Tyr Asn Asp Asp Asp Thr Phe Thr Val Lys Val Gly Asp Lys Glu
        35                  40                  45

Leu Phe Thr Asn Arg Trp Asn Leu Gln Ser Leu Leu Leu Ser Ala Gln
    50                  55                  60

Ile Thr Gly Met Thr Val Thr Ile Lys Thr Asn Ala Cys His Asn Gly
65                  70                  75                  80

Gly Gly Arg Ser Glu Val Ile Phe Arg
                85

<210>  2
<211>  69
<212>  PRT
<213>  Artificial Sequence

<220>
<223>  fragment of toxin subunit B from Shigella dysenteriae

<400>  2

Thr Pro Asp Cys Val Thr Gly Lys Val Glu Tyr Thr Lys Tyr Asn Asp
1               5                   10                  15

Asp Asp Thr Phe Thr Val Lys Val Gly Asp Lys Glu Leu Phe Thr Asn
            20                  25                  30

Arg Trp Asn Leu Gln Ser Leu Leu Leu Ser Ala Gln Ile Thr Gly Met
        35                  40                  45

Thr Val Thr Ile Lys Thr Asn Ala Cys His Asn Gly Gly Gly Arg Ser
    50                  55                  60

Glu Val Ile Phe Arg
65

<210>  3
<211>  15
<212>  PRT
<213>  Artificial Sequence

<220>
<223>  amino acids 43-57 of the HPV 16 E7 protein

<400>  3

Gly Gln Ala Glu Pro Asp Arg Ala His Tyr Asn Ile Val Thr Phe
1               5                   10                  15

<210>  4
<211>  90
<212>  PRT
<213>  Artificial Sequence

<220>
<223>  derivative of toxin subunit B from Shigella dysenteriae

<400>  4

Met Lys Lys Thr Leu Leu Ile Ala Ala Ser Leu Ser Phe Phe Ser Ala
1               5                   10                  15

Ser Ala Leu Ala Thr Pro Asp Cys Val Thr Gly Lys Val Glu Tyr Thr
            20                  25                  30

Lys Tyr Asn Asp Asp Asp Thr Phe Thr Val Lys Val Gly Asp Lys Glu
        35                  40                  45

Leu Phe Thr Asn Arg Trp Asn Leu Gln Ser Leu Leu Leu Ser Ala Gln
    50                  55                  60

Ile Thr Gly Met Thr Val Thr Ile Lys Thr Asn Ala Cys His Asn Gly
65                  70                  75                  80

Gly Gly Arg Ser Glu Val Ile Phe Arg Cys
                85                  90

<210>  5
<211>  70
<212>  PRT

<213>  Artificial Sequence

<220>
<223>  derivative of toxin subunit B from Shigella dysenteriae

<400>  5

Thr Pro Asp Cys Val Thr Gly Lys Val Glu Tyr Thr Lys Tyr Asn Asp
1               5                   10                  15

Asp Asp Thr Phe Thr Val Lys Val Gly Asp Lys Glu Leu Phe Thr Asn
            20                  25                  30

Arg Trp Asn Leu Gln Ser Leu Leu Leu Ser Ala Gln Ile Thr Gly Met
        35                  40                  45

Thr Val Thr Ile Lys Thr Asn Ala Cys His Asn Gly Gly Gly Arg Ser
    50                  55                  60

Glu Val Ile Phe Arg Cys
65                  70

## Description

### FIELD OF THE INVENTION:

The present invention relates to the treatment of cancer and more preferably to HPV-positive cancer. The work leading to this invention has received funding from the European Union Seventh Framework Programme (FP7/2007-2013) under grant agreement n° 304810 and from Canceropole Ile de France (Project HPV-CAN 2011-2013).

### BACKGROUND OF THE INVENTION:

Human Papillomavirus (HPV) is the most common sexually transmitted disease in Western countries. Mucosal infection with HPV types 16 and 18 has long been acknowledged to be a major risk factor for malignant neoplasms such as cervical, anal, penile, vaginal, and head and neck squamous cell carcinoma (HNSCC). The global burden of cancers attributable to HPV infection is estimated at 600,000 cases/year worldwide and accounts for up to 50-80% of oropharyngeal cancers with a steadily increasing rate.

First indications for an involvement of human papillomavirus (HPV) in the etiology of head and neck squamous cell carcinomas (HNSCC) have been obtained in the early 1980s. Subsequently, a specific association of HPV with tumors in Waldeyer's tonsillar ring was found. Research has demonstrated that HPV-positive HNSCC represents a distinct subgroup of malignancies with improved outcomes compared with HPV-negative HNSCC; this is a result of a combination of peculiar biologic features and favorable patient characteristics (younger patients and a minor role of tobacco and alcohol abuse). Since 2000, several larger studies (n>90) confirmed an association between HPV, especially HPV16, and HNSCC, such as oropharyngeal squamous cell carcinomas (OPSCC). Now, both the analysis of viral load and of viral oncogene expression is applied to identify HNSCC with biologically active HPV16, whose oncogenic role is supported by the expression of E6 and E7.

Because of their prognostic, HPV-positive HNSCC patients are more likely to have a durable benefit from radiotherapy, which is now recognized as a valuable option for HNSCC.

Actually, stages I-II HNSCC are treated with a radiation dose depending on tumor size; however, for early stage disease, doses of 66-74 Gy (2.0 Gy/fraction; daily Monday-Friday in 7wk) may be used with adequate results. For later stages III-IV, similar radiotherapy doses are used but in combination with chemotherapy or following surgery.

Because of the increasing incidence of HPV-related cancers and the promising results of radiotherapy, there is a need for the further optimization of radiotherapy treatments by integrating novel strategies to improve radiotherapy's biological efficacy and limit the toxicities related to dose escalation in this subset of patients.

### DETAILED DESCRIPTION OF THE INVENTION:

The inventors established that a combination of a tumor vaccine and of radiotherapy results in a strong synergy leading to the healing of most of the animals, said synergy enabling to halve the radiotherapy dose.

Whereas the inventors established previously that an intranasal immunization with said cancer vaccine was necessary so as to elicit a sustained antitumor response in a murine model of HPV-positive head and neck cancer, they now show that, in combination with radiotherapy, a parenteral administration of said vaccine lead to a strong immune response leading to tumor regression.

The efficiency of said treatment prevents metastasis appearance and also their progression.

Finally, the inventors show that the immunisation with said cancer vaccine may be initiated before irradiation without affecting the efficiency of the treatment.

Thus, in a first aspect, the present invention concerns a composition for use in the prevention or treatment of a subject suffering from a cancer in combination with radiotherapy, wherein said composition comprises a conjugate, eventually associated with pharmaceutically acceptable carrier, said conjugate comprising:
i) the Shiga Toxin B subunit (STxB), a fragment or a derivative thereof; and
ii) at least one epitope associated with said cancer.

As used herein, the term "subject" denotes a mammal, such as a rodent, a feline, a canine or a primate, and most preferably a human.

As used herein an "epitope associated with a cancer" refers to an epitope derived from an antigen expressed in a cancer. Such antigen may be related to tumor neovascularization or to tumor extracellular matrix, or may be a tumoral antigen.

As used herein, an "antigen related to tumor neovascularization" refers to an antigen which is expressed by the neo-synthetized blood vessels present in the tumor.

As an example of such antigen, one can cite the EDA and the EDB domains of fibronectin, Endosalin/TEM1, Endoglin/105, PSMA or B7-H4.

As used herein, an "antigen related to tumor extracellular matrix" refers to an antigen which is expressed in the extracellular matrix present in the tumor.

As an example of such antigen, one can cite the G45 fragment of laminin-332 (ROUSSELLE et al., Cancer Research, vol.68(8), p:2885-94, 2008).

As used herein a "tumoral antigen" refers to an antigenic polypeptide produced in tumor cells. Many tumoral antigens are well known from the skilled person and one can cite, as non limiting examples, CD-20, CEA, EGFR, EPCAM, MUC1, PSMA, CD-19, CAIX, or HER2. When the cancer is an HPV-positive cancer, one may also cite HPV-16 or HPV-18 proteins, such as E6 or E7 proteins.

The Shiga Toxin is a bacterial toxin of the AB₅ subunit family that is secreted by *Shigella dysenteriae.* The A-subunit is the toxic moiety and inhibits the protein synthesis in higher eukaryotic target cells after transferring into the cytoplasm of said cells. The B-subunit is a homopentamer protein (5B - fragments) and is responsible for toxin binding to and internalization into target cells by interacting with the glycolipid Gb3 found on the plasma membranes of these cells. The B-fragment is non toxic, but conserves the intracellular transport characteristics of the holotoxin which, in many Gb3 expressing cells, is transported in a retrograde fashion from the plasma membranes to cytosol, via endosomes. The glycolipid Gb3 receptor enabling said transport, is also reported to be expressed preferentially in some ectodermic derived tumors (plasma) and in some Burkitt's lymphoma, and is also known as CD 77.

As used herein, the term "Shiga Toxin B subunit" or "STxB" corresponds to the polypeptide having the accession number 1410186B and the sequence SEQ id n°1: MKKTLLIAAS LSFFSASALA TPDCVTGKVE YTKYNDDDTF TVKVGDKELF TNRWNLQSLL LSAQITGMTV TIKTNACHNG GGRSEVIFR

As used herein, the term "fragment" refers to a polypeptide having a length of at least 50 amino acids, preferably of at least 60 amino acids, and most preferably of at least 65 amino acids, which fragment binds to the glycolipid Gb3 receptor with a K_{D} equal or inferior to 10⁻⁷ M, preferably equal or inferior to 10⁻⁸ M. Preferably said STxB fragment refers to the sequence SEQ id n°2:
TPDCVTGKVE YTKYNDDDTF TVKVGDKELF TNRWNLQSLL LSAQITGMTV TIKTNACHNG GGRSEVIFR

As used herein, the term "STxB derivatives" refers to an amino acid sequence which fragment binds to the glycolipid Gb3 receptor with a K_{D} equal or inferior to 10⁻⁷ M, preferably equal or inferior to 10⁻⁸ M, and which has a percentage of identity of at least 95 % (i.e. corresponding to about 4 amino acids substitutions) as compared to the amino acid SEQ id n°: 1, preferably of at least 97.5 % (i.e. corresponding to about 2 amino acids substitutions), and more preferably of at least 98.5% (i.e. corresponding to about 1 amino acids substitutions). Such derivatives can be simply identified by the skilled person in view of its personal knowledge and of the teaching of the present patent application. It will also be understood that natural amino acids may be replaced by chemically modified amino acids. Typically, such chemically modified amino acids increase the polypeptide half life.

As used herein, "percentage of identity" between two amino acids sequences, means the percentage of identical amino-acids, between the two sequences to be compared, obtained with the best alignment of said sequences, this percentage being purely statistical and the differences between these two sequences being randomly spread over the amino acids sequences. As used herein, "best alignment" or "optimal alignment", means the alignment for which the determined percentage of identity (see below) is the highest. Sequences comparison between two amino acids sequences are usually realized by comparing these sequences that have been previously aligned according to the best alignment; this comparison is realized on segments of comparison in order to identify and compare the local regions of similarity. The best sequences alignment to perform comparison can be realized, beside by a manual way, by using the global homology algorithm developed by SMITH and WATERMAN (Ad. App. Math., vol.2, p:482, 1981), by using the local homology algorithm developed by NEDDLEMAN and WUNSCH (J. Mol. Biol., vol.48, p:443, 1970), by using the method of similarities developed by PEARSON and LIPMAN (Proc. Natl. Acad. Sci. USA, vol.85, p:2444, 1988), by using computer softwares using such algorithms (GAP, BESTFIT, BLAST P, BLAST N, FASTA, TFASTA in the Wisconsin Genetics software Package, Genetics Computer Group, 575 Science Dr., Madison, WI USA), by using the MUSCLE multiple alignment algorithms (Edgar, Robert C., Nucleic Acids Research, vol. 32, p:1792, 2004 ). To get the best local alignment, one can preferably use the BLAST software with the BLOSUM 62 matrix. The identity percentage between two sequences of amino acids is determined by comparing these two sequences optimally aligned, the amino acids sequences being able to encompass additions or deletions in respect to the reference sequence in order to get the optimal alignment between these two sequences. The percentage of identity is calculated by determining the number of identical position between these two sequences, and dividing this number by the total number of compared positions, and by multiplying the result obtained by 100 to get the percentage of identity between these two sequences.

As used herein, the term "cancer associated epitope" refers to an epitope derived from an antigen related to tumor neovascularization or to tumor extracellular matrix, or against a tumoral antigen.

In a preferred embodiment, the cancer is a HPV-positive cancer, and the epitope associated with said cancer is a HPV epitope, preferably a HPV16 epitope.

As used herein, the term "HPV-positive cancer" refers to a malignant neoplasm associated with a HPV infection, and thus expressing HPV antigen. As an example, such malignant neoplasms may include cervical, anal, penile, vaginal, and head and neck squamous cell carcinoma (HNSCC). Preferably, said HPV-positive cancer is a HPV-positive HNSCC.

Now, said cancer may also correspond to metastasis.

Thus, said HPV-positive cancer may be a metastasis.

As used herein, the term a "HPV16 epitope" refers to HPV16 peptidic sequence, which can be presented by MHC class II. Preferably, said HPV16 epitope is a HPV16 epitope from the E6 or E7 protein of HPV 16. The sequence of the E6 protein from HPV16 is well known from the skilled person. As an example, one can cite the sequence of E6 protein with the accession numbers AHK23256.1, AIQ82784.1, AGM34407.1, ACJ66714.1 or AAO85408.1. The sequence of the E7 protein from HPV16 is also well known from the skilled person. As an example, one can cite the sequence of E7 protein with the accession numbers AHK23257.1, AIQ82815.1, AGM34442.1, ACR25131.1 or ACJ66717.1.

The skilled person can also simply identify the HPV16 epitope among E6 or E7 protein. As an example, one can cite those disclosed in WO 02/090382 or WO2008147187disclosing E6 and E7 HPV16 epitopes, or in WO 02/070006 disclosing the peptide consisting of amino acids 127-142 of HPV16 E6 protein, the peptide consisting of amino acids 35-50 of HPV16 E7 protein, the peptide consisting of amino acids 43-77 of HPV16 E7 protein or the peptide consisting of amino acids 50-62 of HPV 16 E7 protein. One can also cites the epitope disclosed in STRANG et al. (J. Gen. Virol., vol.71, p:423-31, 1990) corresponding to a peptide consisting of amino acids 42-57 of HPV16 E6 protein, or the one disclosed in ALTMANN et al. (Eur. J. Cancer, vol.28, p:326-33, 1992) corresponding to the peptide consisting of amino acids 5-18 of HPV16 E7 protein, the peptide consisting of amino acids 17-34 of HPV 16 E7 protein and the peptide consisting of amino acids 69-82 of HPV 16 E7 protein. One can also cites the peptide consisting of amino acids 43-57 or 49-57 of HPV16 E7 protein disclosed in ADOTEVI et al. (J. Immunol., vol., p: 3371-3379, 2007).

Advantageously, said HPV16 epitope is from HPV16 E7 protein, and is selected in the group comprising the peptides consisting of amino acids 5-18, 17-34, 35-50, 43-57, 43-77, 49-57, 50-62, 69-82 of the HPV 16 E7 protein. Preferably, said HPV16 epitope consists in the amino acids 43-57 of the HPV 16 E7 protein (SEQ id n°3; GQAEPDRAHYNIVTF).

The conjugate comprises a pentamer of STxB, fragment or derivative thereof. In this conjugate, i) the Shiga Toxin B subunit (STxB), a fragment or a derivative thereof; and ii) the at least one HPV16 epitope may be also covalently linked using bifunctional protein coupling agents or in a fusion protein. Naturally, the natural amino acids in said conjugate may be replaced by chemically modified amino acids. Typically, such chemically modified amino acids enable to increase the polypeptide half-life.

In a particular embodiment, i) the Shiga Toxin B subunit (STxB), a fragment or a derivative thereof; and ii) the at least one cancer associated epitope are covalently linked using a bifunctional protein coupling agents.

Bifunctional protein coupling agents are well known from the skilled person such as methods using them, and include, as examples, m-maleimidobenzoyl-N-hydroxysulfosuccinimide ester, N-succinimidyl (2-pyridyldithio) propionate (SPDP), succinimidyl (N-maleimidomethyl) cyclohexane-1-carboxylate, iminothiolane (IT), bifunctional derivatives of imidoesters (such as dimethyl adipimidateHCL), active esters (such as disuccinimidylsuberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6diisocyanate), and bis-active fluorine compounds (such as 1,5-difluoro-2,4- dinitrobenzene).

Preferably, the i) the Shiga Toxin B subunit (STxB), a fragment or a derivative thereof refers to the sequences disclosed in WO 02/060937, wherein a Cysteine residue is added at the C-terminus. As an example, said STxB derivatives refer to the SEQ id n°4 and SEQ id n°5, preferably SEQ id n°5.

| | |
|---|---|
| SEQ id n°4 | MKKTLLIAAS LSFFSASALA TPDCVTGKVE YTKYNDDDTF TVKVGDKELF TNRWNLQSLL LSAQITGMTV TIKTNACHNG GGRSEVIFRC |
| SEQ id n°5 | TPDCVTGKVE YTKYNDDDTF TVKVGDKELF TNRWNLQSLL LSAQITGMTV TIKTNACHNG GGRSEVIFRC |

In a particular embodiment, i) the Shiga Toxin B subunit (STxB), a fragment or a derivative thereof; and ii) the at least one cancer associated epitope are covalently linked in a fusion protein.

The term "fusion protein", also known as a chimeric protein, refers to a protein created through the joining of i) the Shiga Toxin B subunit (STxB), a fragment or a derivative thereof; and ii) the at least one cancer associated epitope, which are originally coded for separate polypeptides.

Both sequences may be separated or not by a "linker" amino acid sequence.

In a particular embodiment, the sequences i) and ii) are not separated by any inker such as for the conjugate disclosed in WO 99/03881.

Said "linker" amino acid sequence may be of a length sufficient to ensure that the fusion protein form proper secondary and tertiary structures. The length of the linker amino acid sequence may vary without significantly affecting the biological activity of the fusion protein. Typically, the length of the linker is comprised between 5 and 30 amino acids, preferably between 10 and 30 amino acids, more preferably between 15 and 30 amino acids.

Preferred linker amino acid sequences are those which allow the conjugate to adopt a proper conformation. The most suitable linker amino acid sequences (1) will adopt a flexible extended conformation, (2) will not exhibit a propensity for developing ordered secondary structure which could interact with the functional domains of fusion proteins, and (3) will have minimal hydrophobic or charged character which could promote interaction with the functional protein domains. Preferably, the linker amino acid sequence comprises near neutral amino acids selected in the group comprising Gly (G), Asn (N), Ser (S), Thr (T), Ala (A), Leu (L), and Gln (Q), most preferably in the group comprising Gly (G), Asn (N), and Ser (S). Examples of linker sequences are described in U.S. Pat. Nos. 5,073,627 and 5,108,910.

The composition comprises an "effective amount" of the conjugate, which effective amount is sufficient to induce an immune response against the at least one cancer associated epitope, preferably sufficient to induce the regression of tumor growth, notably in combination with radiotherapy. The doses used for the administration can be adapted as a function of various parameters, in particular as a function of the mode of administration used, of the relevant pathology, or alternatively of the desired duration of treatment. Naturally, the form of the pharmaceutical composition, the route of administration, the dosage and the regimen naturally depend on the condition to be treated, the severity of the illness, the age, weight, and sex of the subject, etc. The ranges of effective doses provided below are not intended to limit the invention and represent preferred dose ranges. However, the preferred dose can be tailored to the individual subject, as is understood and determinable by one of skill in the art, without undue experimentation.

In view of the marked efficiency of said conjugate, the skilled person can plan to use small doses for treating a subject suffering from a cancer. As a non limiting example, the conjugate can be parenterally administered by injection at a dose in a range between 10 µg and 1000 µg, preferably in a range between 100 µg and 300 µg Now, said conjugate administration may be done once, or repeated one or several time during the treatment of the subject.

Radiation therapy or radiotherapy is the medical use of irradiation -i.e. ionizing radiation- as part of cancer treatment to control malignant cells. It is used as palliative treatment or as therapeutic treatment. Radiotherapy is accepted as an important standard therapy for treating various types of cancers.

As used herein, the term "radiotherapy" is used for the treatment of diseases of oncological nature with irradiation corresponding to ionizing radiation. Ionizing radiation deposits energy that injures or destroys cells in the area being treated (the target tissue) by damaging their genetic material, making it impossible for these cells to continue to grow. Radiotherapy may be used to treat localized solid tumors cancers of the skin, tongue, larynx, brain, breast, lung or uterine cervix. One type of radiation therapy commonly used involves photons, e.g. X-rays. Depending on the amount of energy they possess, the rays can be used to destroy cancer cells on the surface of or deeper in the body. The higher the energy of the x-ray beam, the deeper the x-rays can go into the target tissue. Linear accelerators and betatrons produce x-rays of increasingly greater energy. The use of machines to focus radiation (such as x-rays) on a cancer site is called external beam radiotherapy. Gamma rays are another form of photons used in radiotherapy. Gamma rays are produced spontaneously as certain elements (such as radium, uranium, and cobalt 60) release radiation as they decompose, or decay.

Another technique for delivering radiation to cancer cells is to place radioactive implants directly in a tumor or body cavity. This is called internal radiotherapy.

Brachytherapy, interstitial irradiation, and intracavitary irradiation are types of internal radiotherapy. In this treatment, the radiation dose is concentrated in a small area, and the patient stays in the hospital for a few days. Internal radiotherapy is frequently used for cancers of the tongue, uterus, and cervix.

A further technique is intra-operative irradiation, in which a large dose of external radiation is directed at the tumor and surrounding tissue during surgery.

Another approach is particle beam radiation therapy. This type of therapy differs from photon radiotherapy in that it involves the use of fast-moving subatomic particles to treat localized cancers. Some particles (neutrons, pions, and heavy ions) deposit more energy along the path they take through tissue than do x-rays or gamma rays, thus causing more damage to the cells they hit. This type of radiation is often referred to as high linear energy transfer (high LET) radiation. Radio-sensitizers make the tumor cells more likely to be damaged, and radio-protectors protect normal tissues from the effects of radiation.

A person of ordinary skill in the radiotherapy art knows how to determine an appropriate dosing and application schedule, depending on the nature of the disease and the constitution of the patient. In particular, the person knows how to assess dose-limiting toxicity (DLT) and how to determine the maximum tolerated dose (MTD) accordingly.

More particularly, the amount of radiation used in photon radiation therapy is measured in gray (Gy), and varies depending on the type and stage of cancer being treated. For curative cases, the typical dose for a solid epithelial tumor ranges from 60 to 80 Gy. Many other factors are considered by radiation oncologists when selecting a dose, including whether the patient is receiving chemotherapy, patient co-morbidities, whether radiation therapy is being administered before or after surgery, and the degree of success of surgery. Moreover, the total dose is often fractionated (spread out over time) for several important reasons. In fact, fractionation allows normal cells time to recover, while tumor cells are generally less efficient in repair between fractions. Fractionation also allows tumor cells that were in a relatively radioresistant phase of the cell cycle during one treatment to cycle into a sensitive phase of the cycle before the next fraction is given. Similarly, tumor cells that were chronically or acutely hypoxic (and therefore more radioresistant) may reoxygenate between fractions, improving the tumor cell kill. Fractionation regimes are individualized between different radiotherapy centers and even between individual doctors. In North America, Australia, and Europe, the typical fractionation schedule for adults is 1.8 to 2 Gy per day, five days a week. In some cancer types, prolongation of the fraction schedule over too long can allow for the tumor to begin repopulating, and for these tumor types, including head-and-neck and cervical squamous cell cancers, radiation treatment is preferably completed within a certain amount of time. In some cases, two fractions per day are used near the end of a course of treatment. This schedule, known as a concomitant boost regimen or hyperfractionation, is used on tumors that regenerate more quickly when they are smaller. In particular, tumors in the head-and-neck demonstrate this behavior.

The inventors have established that the combined treatment with the conjugate and radiotherapy enables to halve the radiotherapy doses for obtaining a healing.

Thus, in another preferred embodiment, and because of the efficiency of the method of the invention, the radiotherapy can be applied at a dose in a range from about 10 to 55Gy, preferably from about 15 to 50 Gy, such as 20 to 40Gy, , concretely from about 20 to 35 Gy, and more concretely from about 25 to 30 Gy.

As used herein, the term "in combination" refers to the use of more than one therapeutic agent, radiotherapy and the conjugate in the present case. The use of the term "in combination" does not restrict the order in which said therapeutic agents are administered to the subject. A first therapeutic agent, such as a conjugate, can be administered prior to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks before), concomitantly with, or subsequent to (e.g. , 5 minutes, 15 minutes, 30 minutes, 45 minutes, 1 hour, 2 hours, 4 hours, 6 hours, 12 hours, 24 hours, 48 hours, 72 hours, 96 hours, 1 week, 2 weeks, 3 weeks, 4 weeks, 5 weeks, 6 weeks, 8 weeks, or 12 weeks after) the administration of radiotherapy, to a subject with cancer.

Thus, the composition and the radiotherapy may be administrated currently, separately, or sequentially.

The inventors have established that the combination is very efficient by administrating the first dose of the vaccine conjugate prior irradiation. This approach will facilitate the implementation of vaccinations in future clinical strategies since patients with a confirmed diagnosis of HPV-positive cancer would immediately benefit from the vaccination administration while waiting for radiotherapy treatment.

Thus, in a third preferred embodiment, the composition is administrated before the irradiation, preferably at a timing of 1 minute to 14 days before irradiation, most preferably 3 to 7 days before irradiation. Accordingly, said composition is administrated in a subject who has not received radiotherapy, preferably who has not received irradiation since at least 1 minute to 14 days, preferably at least 1 to 3 days.

The expression "pharmaceutically acceptable" refers to molecular entities and compositions that are physiologically tolerable and do not typically produce allergic or similar undesirable reactions, such as gastric upset, dizziness and the like when administered to a human. Preferably, as used herein, the expression "pharmaceutically acceptable" means approvable by a regulatory agency of the Federal or state government or listed in the U.S. Pharmacopeia or other generally recognized pharmacopeia for use in animals, and more particularly in humans.

The term "carrier" refers to a solvent, adjuvant, excipient, or vehicle with which the compound is administered. Such pharmaceutical carriers can be sterile liquids, such as water and oils, including those of petroleum, animal, vegetable or synthetic origin, such as peanut oil, soybean oil, mineral oil, sesame oil and the like.

The conjugate may be solubilized in a buffer or water or incorporated in emulsions, microemulsions, hydro gels (e.g. PLGA-PEG-PLGA triblock copolymers-based hydrogels), in microspheres, in nanospheres, in microparticles, in nanoparticles (e.g. poly(lactic-co-glycolic acid) microparticles (e.g. poly lactic acid (PLA) ; poly (lactide-co-glycolic acid) (PLGA) ; polyglutamate microspheres, nanospheres, microparticles or nanoparticles), in liposomes, or other galenic formulations. In all cases, the formulation must be sterile and fluid to the extent of acceptable syringability. It must be stable under the conditions of manufacture and storage and must be preserved against the contaminating action of microorganisms, such as bacteria and fungi.

Dispersions can also be prepared in glycerol, liquid polyethylene glycols, and mixtures thereof and in oils. Under ordinary conditions of storage and use, these preparations contain a preservative to prevent the growth of microorganisms.

The conjugate can be formulated into a composition in a neutral or salt form. Pharmaceutically acceptable salts include the acid addition salts (formed with the free amino groups of the protein) which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like.

The carrier can also be a solvent or a dispersion medium containing, for example, water, ethanol, polyol (for example, glycerol, propylene glycol, and liquid polyethylene glycol, and the like), suitable mixtures thereof, and vegetables oils. The conjugates of the invention may also be modified, by pegylation as an example, so as to increase its biodisponibility.

The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride.

Prolonged absorption of the injectable compositions can be brought about by the use in the compositions of agents delaying absorption, for example, aluminium monostearate, gelatin, polyols, and half-life enhancing covalent and non covalent formulations.

There are numerous causes of peptide instability or degradation, including hydrolysis and denaturation. Hydrophobic interaction may cause clumping of molecules together (i.e. aggregation). Stabilizers may be added to reduce or prevent such problems.

Stabilizers include cyclodextrine and derivatives thereof (see U.S. Pat. No.5,730,969). Suitable preservatives such as sucrose, mannitol, sorbitol, trehalose, dextran and glycerin can also be added to stabilize the final formulation. A stabilizer selected from ionic and non-ionic surfactants, D-glucose, D-galactose, D-xylose, D-galacturonic acid, trehalose, dextrans, hydroxyethyl starches, and mixtures thereof may be added to the formulation. Addition of alkali metal salt or magnesium chloride may stabilize a peptide. The peptide may also be stabilized by contacting it with a saccharide selected from the group consisting of dextran, chondroitin sulphuric acid, starch, glycogen, dextrin, and alginic acid salt. Other sugars that can be added include monosaccharides, disaccharides, sugar alcohols, and mixtures thereof (E.g., glucose, mannose, galactose, fructose, sucrose, maltose, lactose, mannitol, xylitol). Polyols may stabilize a peptide, and are water-miscible or water-soluble. Suitable polyols may be polyhydroxy alcohols, monosaccharides and disaccharides including mannitol, glycerol, ethylene glycol, propylene glycol, trimethyl glycol, vinyl pyrrolidone, glucose, fructose, arabinose, mannose, maltose, sucrose, and polymers thereof. Various excipients may also stabilize peptides, including serum albumin, amino acids, heparin, fatty acids and phospholipids, surfactants, metals, polyols, reducing agents, metal chelating agents, polyvinyl pyrrolidone, hydrolysed gelatin, and ammonium sulfate.

The composition may be formulated for injection, e.g. local injection, transmucosal administration (i.e. intranasal...), inhalation, oral administration and more generally any formulation that the skilled person finds appropriate to achieve the desired therapy.

Now, whereas the inventors have priory established that the HPV vaccine induces sufficient immune response only by intranasal administration; they now show that a parenteral administration in combination with radiotherapy induces a sufficient immune response so as to destroy the target cancer.

Thus, in still another preferred embodiment, the composition is for use in the prevention or treatment of a subject suffering from a cancer attributable to HPV infection by a parenteral administration.

As used herein, the term "parenteral" includes intravenous, intramuscular, subcutaneous, rectal, vaginal or intraperitoneal administration.

Thus, the used composition contains vehicles which are pharmaceutically acceptable for a formulation intended to be injected. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions. Suitable pharmaceutical carriers are described in "Remington's Pharmaceutical Sciences" by E.W. Martin.

In another aspect, the present invention relates to a method for treating or preventing cancer in a subject comprising the step of administrating to said subject a composition as described previously in combination with radiotherapy.

In the context of the invention, the term "treating" or "treatment", as used herein, means reversing, alleviating, inhibiting the progress of, or preventing the disorder or condition to which such term applies, or one or more symptoms of such disorder or condition. The term "treating cancer" as used herein means the inhibition of the growth of cancer cells. Preferably such treatment also leads to the regression of tumor growth, i.e., the decrease in size of a measurable tumor. Most preferably, such treatment leads to the complete regression of the tumor.

Other embodiments and advantages of the present invention are illustrated in the following non-limiting examples.

### EXAMPLES

### 1) StTxB-E7 Vaccine Production:

The StTxB-E7 vaccine was produced by the chemical coupling of the N-bromoacetylated E7₄₃₋₅₇ peptide (SEQ id n°...; GQAEPDRAHYNIVTF) and the sulfhydryl group of the recombinant nontoxic B subunit of Shiga toxin (SEQ id n°...) as disclosed in HIACHEUR et al. (Journal of immunology, vol.165(6), p:3301-8, 2000).

### 2) Head and neck tumor and lung metastasis models

Female C57BL/6 mice (age, 7 to 8 weeks) were purchased from JANVIER CERT and housed in the GUSTAVE ROUSSY animal facility. All animal procedures were performed according to the protocols approved by the Ethical Committee CEEA 26 and in accordance with recommendations for the proper use and care of laboratory animals.

To establish a head and neck tumor implantation model, TC1/Luc cells were used. These firefly luciferase-expressing TC1/Luc cells, generated by the HPV16 E6/E7 and c-H-ras retroviral transduction of lung epithelial cells of C57BL/6 origin, were kindly provided by T.C. WU (Johns Hopkins Medical Institutions, Baltimore, MD, USA). The cells were cultured *in vitro* in RPMI 1640 supplemented with 10% fetal bovine serum, 1% sodium pyruvate, 1% non-essential amino acids and 10 mM HEPES and were grown at 37°C with 5% CO2. Then, syngeneic tumor grafts were initiated by injection (Day 0) of 0.1 ml of PBS suspension containing 5× 10⁵ TC1/Luc cells at a submucosal site of the right inner lip of the mice. For rechallenge experiments, mice were injected in the left inner lip. Lung metastases were established by injecting 10⁶ TC1/Luc cells suspended in 0.2 ml of PBS in the tail vein.

### 3) Radiotherapy potentialization using STxB-E7 vaccine

The mice were then subjected to the therapeutic treatments depicted in the schematic of figure 1, with 10 mice per group. Briefly, the mucosal adjuvant α-galactosylceramide (KRN7000, FUNAKOSHI, 20 µg per mouse) was administered intraperitonealy at Day 3 alone or together with the first dose of vaccine STxB-E7 (1 µg per mouse). Then, the mice received or not a single-beam local irradiation to the head and neck region using a 200 kV Varian X-ray irradiator. This selective irradiation of the tumor grafts was performed by the interposition of a 4-cm-thick lead shield and corresponded to a single fraction 7.5 Gy. The KRN7000-treated mice were used as control groups since KRN7000, either alone or in combination with radiotherapy, did not impact tumor growth (data not shown).

To monitor tumor growth, bioluminescence imaging was performed using the XENOGEN *IN VIVO* IMAGING SYSTEM 50 (IVIS; CALIPER LIFE SCIENCES). Mice were placed in the supine position in the imaging chamber 5 minutes after intraperitoneal injection of firefly luciferin; images were acquired over a 15- to 30-second period while the mice were under sedation via the continuous administration of isoflurane. The photographic images and bioluminescence color images were superimposed, and signal quantification was performed using the LIVINGIMAGE V 4.3.1 software (CALIPER LIFE SCIENCES). The bioluminescent signal as a measure of tumor size was analyzed using an ANOVA analysis followed by Tukey's post-hoc test for multiple comparisons. Simultaneously, the survival data were analyzed using the Kaplan-Meier and Log-rank tests for survival distribution. Correlations were assessed using Pearson's correlation test, and statistical analyses were performed using PRISM Version 6 (GRAPHPAD).

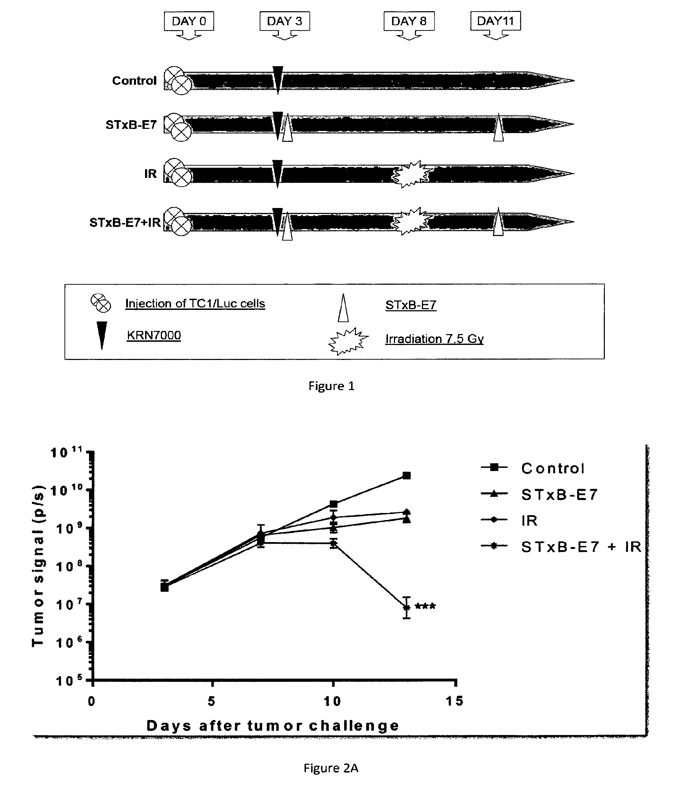

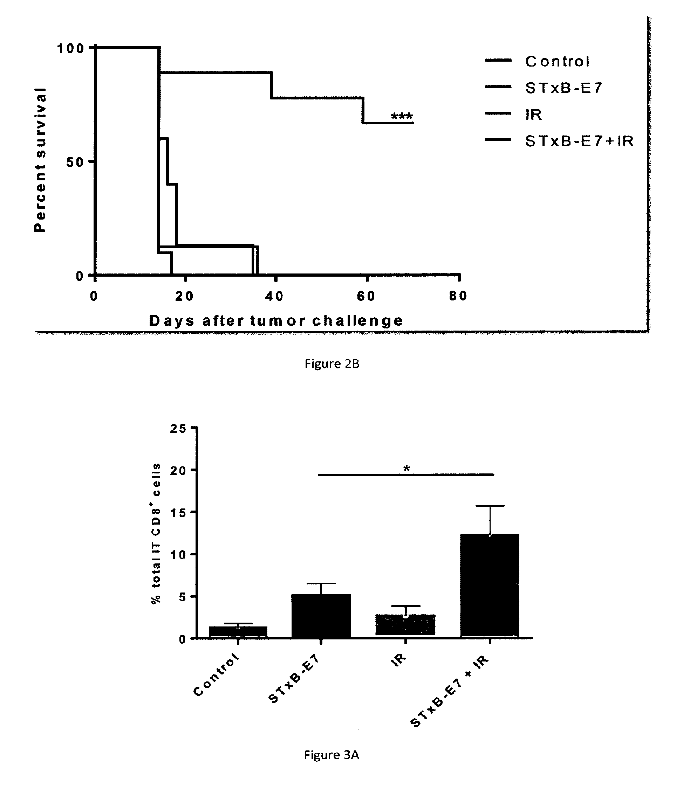

The figure 2A shows the tumor signal as determined by *in vivo* bioluminescent imaging following TC1/Luc cells mouse injection with time, and the figure 2B shows (***p<0.001) the Kaplan-Meier survival curves for the different groups.

The bioluminescent *in vivo* imaging results showed that the mice developed fast-growing tumors (Fig. 2A) that resulted in the survival of control mice for only two weeks after TC1/Luc cell injection (Fig. 2B). Treatment with a single dose of 7.5 Gy IR resulted in a limited tumor growth delay and a non-significant enhanced survival (Fig. 2B).

The systemic immunization with STxB-E7 only partially increased tumor control, whereas its combination with local radiotherapy led to an effective antitumor response with 70% of the mice still alive 70 days after the tumor graft (Fig. 2B, p<0.001 *vs* control). Moreover, the tumor volume of mice treated with the combined therapy was significantly smaller compared with the one of single therapy (IR or STxB-E7)-treated mice (Fig. 2A, p<0.01, one-way ANOVA). Finally, most of the mice in the combination therapy group had complete tumor clearance that lasted until the end of the 90-day observation period (data not shown). Of note, when the tumors were irradiated with a 10.4 Gy dose in 4 consecutive fractions (2.6 Gy/day), representing the biologically equivalent dose (BED) of 7.5 Gy in a single fraction as calculated using the linear quadratic model with an α/β=10, the results were similar (data not shown).

These data indicate that the novel therapeutic approach based on the combination of STxB-E7 with radiotherapy resulted in the complete clearance of the majority of treated tumors in a relevant model of HNSCC, even after an 18-month observation period. This new radiovaccination strategy acts synergistically with either single dose or fractionated IR, which is of particular relevance because fractionated radiotherapy is the mainstay for the treatment of HNSCC. Moreover, the possibility to use low irradiation doses could be a valuable tool for treatment deintensification strategies in the clinic, since it can limit the risk of late complications.

### 4) tumor-infiltrating and splenic antigen-specific CD8⁺ T cells induced by combined irradiation and vaccination

Since CD8⁺ T cell population play a pivotal role in the tumor control of TC1, the presence of anti-E7₄₉₋₅₇/D^{b}-specific CD8⁺ T cells in tumors was analyzed.

For this analysis, 4 groups of 5 mice were treated as described previously and their tumors and spleen were then dissociated at day 14, washed twice in PBS and then incubated with the Fc receptor block CD16/CD32 (EBIOSCIENCE). The tumors were then labeled with the E7₄₉₋₅₇/D^{b} tetramer according to the manufacturer's recommendations (BECKMAN COULTER IMMUNOMICS) and the anti-CD8 antibody. Cells were incubated for 35 minutes at 4°C with the PE-labeled tetramer. After incubation and washes, the cells were labeled using the Live/Dead cell viability assay (LIFE TECHNOLOGIES) and anti-CD8 (APC-Cy7, EBIOSCIENCE), PD1 (FITC, EBIOSCIENCE), CTLA4 (Brilliant Violet 421, BIOLEGEND), and Tim3 (APC, EBIOSCIENCE) mAbs for 20 minutes to phenotype the tetramer-positive CD8⁺ T cells. Irrelevant tetramers recognizing a LCMV derived peptide in the context of D^{b} molecules were used. CD8⁺ cells were detected by flow cytometry and expressed as percentage of total living cells using the FLOWJO (TREE STAR) software.

The figures 3A and 3B show the E7-specific CD8⁺ T cells detected intratumorally as expressed as the percentage of total CD8+ cells and the percentage of H-2Db E7-tetramer per CD8⁺T cells.

The figure 4 shows percentage of H-2Db E7-tetramer per CD8⁺T cells in the spleen of said mice.

The figure 5 shows the tumor sizes obtained by IVIS *in vivo* imaging at the day of sacrifice plotted against the percentage of tumor infiltrating E7-specific CD8⁺. *p<0.05; one-way ANOVA followed by Tukey's post-test.

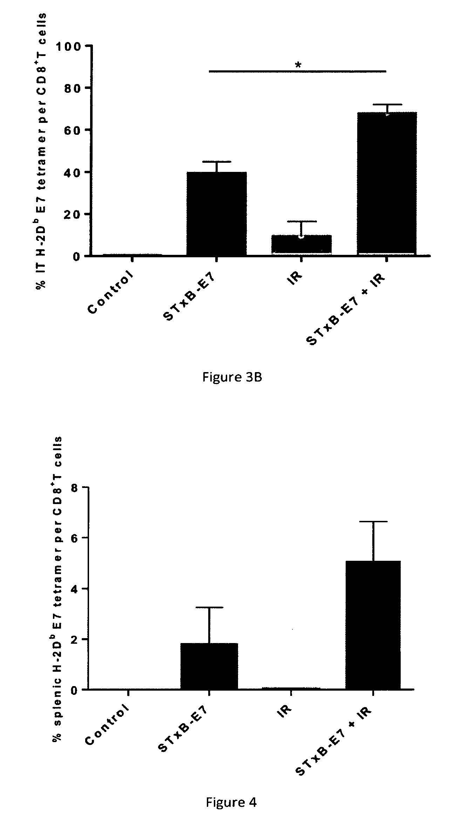

The results show that the total intratumor CD8⁺ levels were low in control tumors, and almost no E7-specific CD8⁺ T cells were found in this group (Fig. 3). Irradiation alone was not sufficient to alter the CD8⁺ infiltrate, whereas vaccination upregulated the percentage of E7₄₉₋₅₇ specific CD8⁺ T cells, as demonstrated by the E7-tetramer analysis. When IR was added to STxB-E7 immunization, the infiltration of CD8⁺ T cells was significantly boosted (Fig. 3A). Most of the CD8⁺ T cells were antigen-specific, as the level of E7 tetramer-positive CD8⁺ T lymphocytes reached a surprising 67% in this group (Fig. 3B).

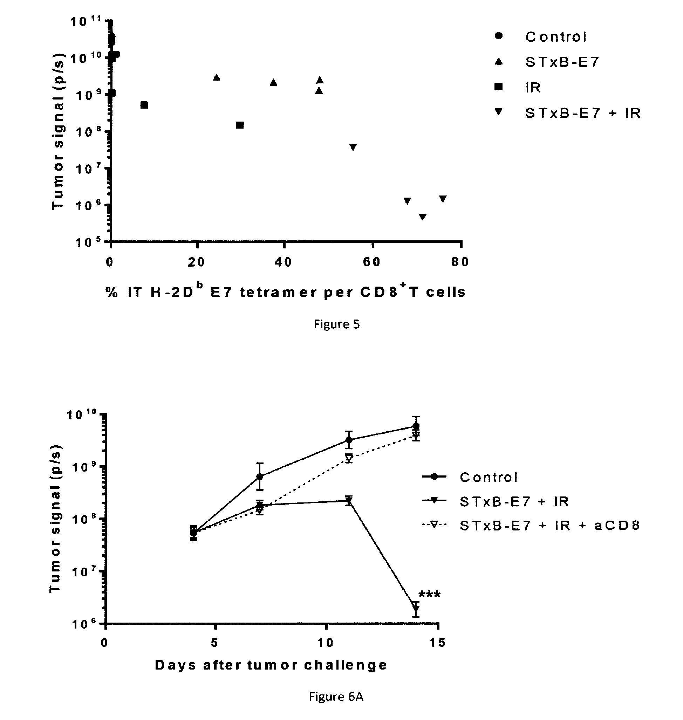

The qualitative analysis of the E7-specific CD8⁺ infiltrate by identifying the PD1, CTLA4 and Tim3 positive subpopulations does not reveal any significant differences among the treatment groups (data not shown). Now, the induction of a specific CD8⁺ T cell response was not restricted to the tumor but was instead systemic. Indeed, the combination of IR and vaccination resulted in the highest antigen-directed response compared with vaccine treatment alone (Fig. 4). Moreover, the level of E7-specific CD8⁺ lymphocytes was inversely correlated with the tumor size (p<0.05, Pearson's correlation test, Fig. 5), suggesting a central role of CD8⁺ T lymphocytes in the antitumor response in the preclinical HNSCC tumor model.

To mechanistically validate the role of CD8⁺ T cells in this therapeutic setting, mice undergoing combined irradiation and vaccination were injected intraperitonealy with 100 µg per mouse of anti-CD8 mAb (clone 2.43; BIOXCELL) or isotype control mAb on day 7 (one day prior to irradiation) to deplete CD8⁺ T cells, as previously reported (SANDOVAL et al., Science translational medicine, vol.5(172), p:172ra20, 2013).

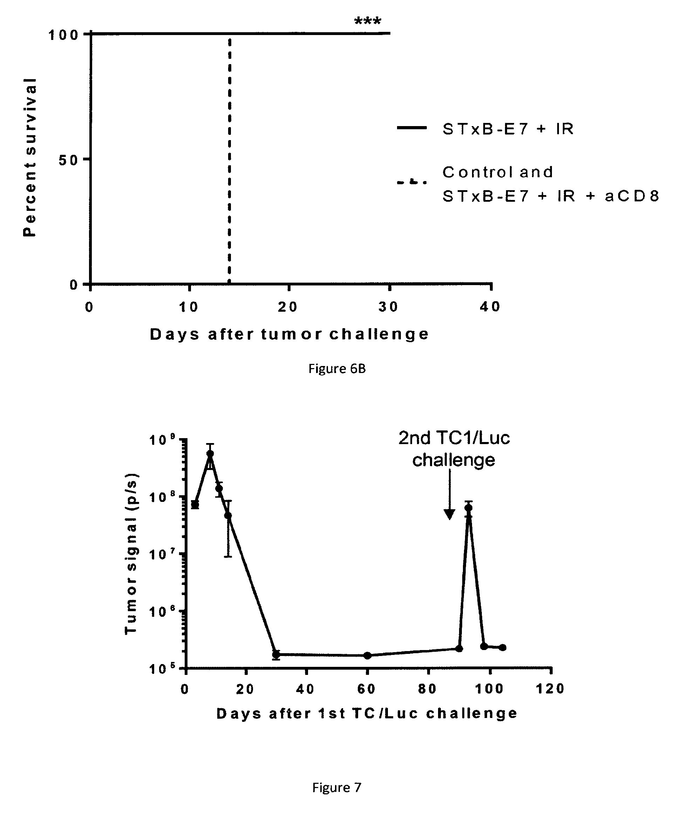

The figures 6A and B show the *in vivo* bioluminescent imaging and the Kaplan curves respectively the mice (N=5) injected with anti-CD8 mAb or isotype control mAb on day 7 (one day prior to irradiation) to deplete CD8⁺ T cells (***p<0.001, one-way ANOVA followed by Tukey's post-test of tumor sizes at day 14). One of two representative experiments is shown.

The results show that the administration of anti-CD8 mAbs has a dramatic effect on the antitumor response, resulting in a complete loss of efficacy of the combined treatment on tumor size and survival, as all of the CD8⁺-depleted mice had to be euthanized together with the controls (Fig. 6A and B).

These data clearly indicate that CD8⁺ T lymphocytes are required to elicit the antitumor response triggered by the combination of irradiation and vaccination.

### 5) T cell memory and STxB-E7/Radiotherapy combination:

Because most of the mice that received the combination of STxB-E7 and IR surprisingly showed a complete remission of the tumor (Fig. 2B), it was wondered whether the sustained CD8⁺ T cell response observed in this group resulted in the development of an effective T cell memory.

To test this hypothesis, four mice that were still tumor free 90 days after tumor grafting, as observed by *in vivo* imaging as previously, were challenged again with TC1/Luc cells in the submucosa of the opposite inner lip; the opposite side was used to avoid potential bias due to local response. It was confirmed that four days after the second injection, all mice were correctly engrafted with tumor cells localized on left inner lip.

The figure 7 shows the results of *in vivo* bioluminescent imaging establishing the tumor signal following the first and second TC1/Luc challenge.

The results show that five days following the second TC1/Luc challenge, and without any additional treatment, the luciferase activity was completely absent, indicating a spontaneous clearance of the tumor cells. In contrast, treatment-naive mice used as positive controls displayed a tumor growth curve similar to those depicted previously (data not shown). When mice were euthanized to collect the spleens 14 days after the second TC1/Luc challenge, the same results were observed (data not shown). Cytofluorimetric analysis showed that rechallenged mice displayed high percentages of E7-specific CD8⁺ T cells at similar levels to those of mice at the end of the combined treatment (data not shown). Finally, a similar experiment was performed on three mice that were rechallenged with TC1/Luc cells 18 months after the first treatment, again resulting in the complete spontaneous rejection of tumor cells (data not shown).

These data indicate that a combination of local irradiation and immunization elicits a CD8⁺ T cell memory that is sufficient to exert a complete antitumor response.

This strong antitumor response may also be explained by the fact that in contrast to other studies, the combination of low doses of irradiation and the cancer vaccine used here did not result in an increase in immunosuppressive cells in the tumor microenvironment (data not shown).

### 6) Metastasis formation and STxB-E7/Radiotherapy combination:

It was also important to evaluate whether the immune response elicited from effective combined therapy provided protection against potential metastasis formation.

This was tested this by intravenously injecting TC1/Luc cells to induce the development of lung metastases. The metastasis presence was confirmed by *in vivo* imaging analysis showing that, in treatment-naive mice, a bioluminescent signal localized to the lungs was already detectable four days after the injection of TC1/Luc cells (data not shown). The size of the lung metastases increased quickly until day 15, when the mice had to be euthanized.

For this evaluation, the results have shown that, in mice that had already experienced a complete remission of the head and neck tumor upon administration of the combination of STxB-E7 and IR (rechallenge group), the tumor signal in the lungs was already smaller at day 4 when compared to treatment-naive mice. TC1/Luc cells were spontaneously cleared starting at day 8 and all four tested mice remained metastasis-free until the end of the 60-day observation period (data not shown).

These data indicate that the combination of local irradiation and immunization is sufficient to exert a complete antitumor response not only on local recurrences, but also on distant metastases.

Consequently, this combination strategy represents a promising clinical tool, since a patient treated with this combination therapy who undergoes tumor remission will likely be protected against any local or distant relapse.

### 7) Tumor vessel remodelling and tumor perfusion

The tumor vascular structure and its activation were assessed in the different treatment groups by immunohistochemistry (IHC). For this, tumors were sampled 9 or 14 days after irradiation. These tumors were then included in OCT for further analysis.

Then, detection on tumor slides of alpha smooth muscle actin (αSMA) was performed using a validated standard protocol on a VENTANA DISCOVERY ULTRA autostainer (ROCHE TISSUE DIAGNOSTICS, primary Ab ref ab5694, ABCAM) according to manufacturers' instructions. Manual staining protocols on OCT frozen sections were used to detect CD31 (BD PHARMINGEN), and ICAM-1 (ABCAM). All the slides were counterstained with hematoxylin. NG2 (primary Ab: MILLIPORE) was detected by immunofluorescence. CD31 and αSMA staining were then quantified using the algorithm for vessel detection in the Tissue Studio software package from DEFINIENS. Vessels were automatically detected according to their IHC staining spectral properties in regions of interest that had been manually selected. The percentage of the area stained for NG2 was measured using the IMAGEJ V1.41 software. ICAM1 staining was determined using a semi-quantitative score based on the staining area (less than 1 per field=0; 1-2 per field=1; 3 or more per field=2) and intensity (no staining=0; weak=1; moderate=2; strong=3).

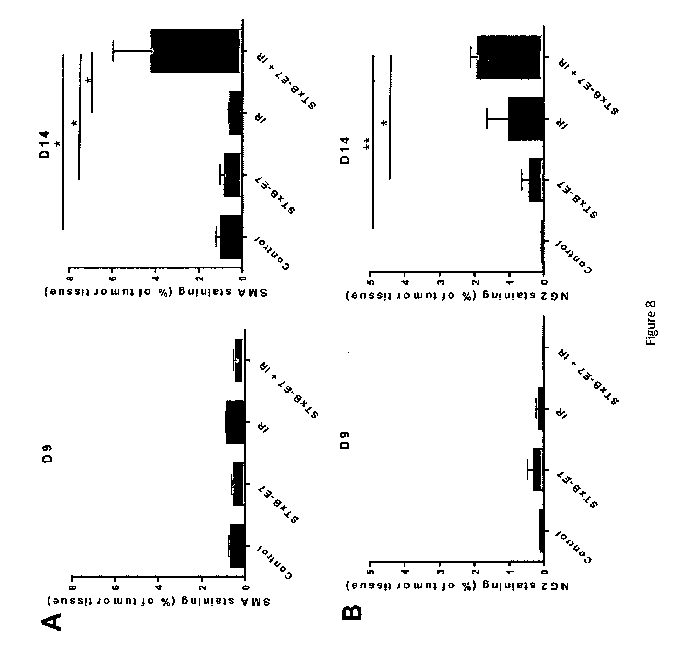

The figure 8 shows the quantification of the antibody stained area for αSMA (A) or NG2 (B) in the tumor sampled at day 9 (D9) or 14 (D14). *p<0.05; **p<0.01 one-way ANOVA followed by Tukey's post-test.

In view of the immunohistochemical staining obtained with CD31, the results established that there were no significant differences in tumor vessel surface and density elicited by the various treatments at either 24 h (day 9) or 6 days after irradiation (day 14) data not shown). In contrast, when the levels of pericytes were analyzed by performing αSMA staining, a significant increase in the stained surface in the tumors of mice treated with both STxB-E7 and IR we observed at day 14 (Fig. 8A). Because pericytes are recognized to be key regulators of the vascular structure and because the extent of their coverage on tumor vessels is typically diminished compared with normal tissues, these data suggest that the combined treatment induces a restoration of vascular functionality. This hypothesis was further confirmed by the increase in NG2 staining (an alternative pericyte marker) observed at day 14 in mice treated with both local irradiation and vaccination (Fig. 8B).

To assess vascular permeability, 0.2 ml Evans Blue dye (0.5%) was administered to mice by tail vein injection. Mice were sacrificed 30 minutes after said injection. Dissected tumor samples were collected, and Evans Blue was eluted in formaldehyde (0.5 ml for 50 mg of tissue) at 58° C overnight. The concentration of the accumulated dye for each aliquot was quantified by the use of a spectrophotometer by reading the absorbance at 610 nm with subtraction of the reference absorbance at 450 nm.

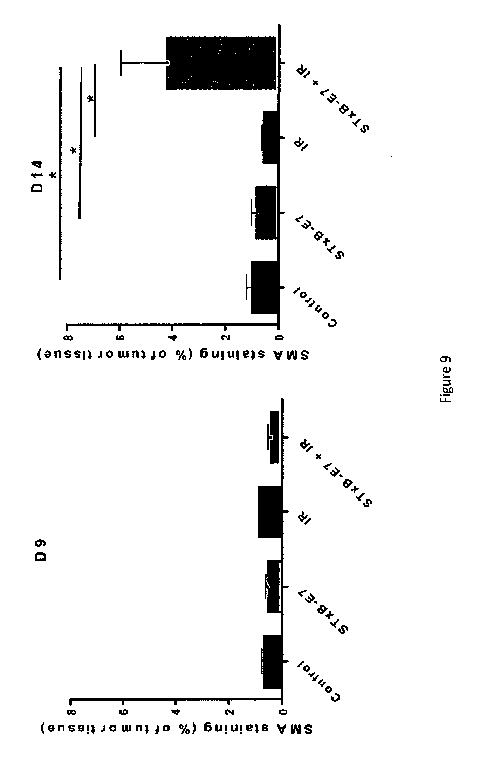

The figure 9 shows the mean ± SEM absorbance related to mean tumor perfusions depending on their treatment at day 9 (D9) or 14 (D14). *p<0.05 one-way ANOVA followed by Tukey's post-test.

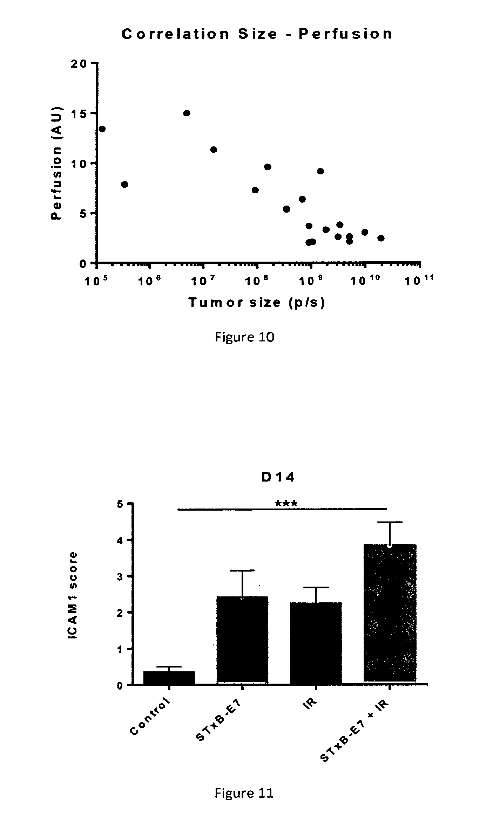

The figure 10 illustrates the data obtained by IVIS *in vivo* imaging at the day of sacrifice plotted against absorbance at day 14 for individual mice to show the correlation between tumor perfusion and size (p<0.001, Pearson correlation test).

These observations were supported by the Miles assay, a functional test that is performed to evaluate tumor perfusion. At 24 hours post irradiation (day 9), the perfusion of tumors was not yet affected by the treatments (Fig. 9, left panel); however, at day 14 after tumor challenge, tumor perfusion was significantly increased only by the combined treatment compared with both control and single therapies (Fig. 9, right panel). This observed increase was inversely correlated with the bioluminescent signal (p<0.05, Pearson's correlation test, Fig. 10), indicating that perfusion was linked to the tumor size at this time point.

Finally, vessel activation was evaluated by detecting ICAM-1 expression in the tumors by IHC. ICAM-1 is a transmembrane protein implied in the arrest and transmigration of leukocytes from blood vessels, which is upregulated in tumors from mice undergoing the different treatments.

The figure 11 shows the quantification of ICAM-1 expression based on a score from immunohistochemical staining. ***p<0.001 one-way ANOVA followed by Tukey's post-test.

The results show that ICAM1 is mainly upregulated in the combination group (Fig. 11).

Taken together, these data indicate that the combination of STxB-E7 immunization and IR promotes tumor vessel normalization and activation, thus favoring the recruitment and infiltration of leukocytes in the tumor tissue. Thus, for the first, a combination therapy based on local irradiation and vaccination induces increased tumor perfusion, which is accompanied by augmented pericyte coverage and ICAM-1 expression.

## Claims

1. A composition for use in the prevention or treatment of a subject suffering from a cancer in combination with radiotherapy, wherein said composition comprises a conjugate, eventually associated with pharmaceutically acceptable carrier, said conjugate comprising:
i) the Shiga Toxin B subunit (STxB), a fragment or a derivative thereof; and
ii) at least one epitope associated with said cancer.

2. The composition of claim 1, wherein the cancer is a HPV-positive cancer and the at least one cancer associated epitope is a HPV epitope.

3. The composition of claim 2, wherein the HPV-positive cancer is a HPV-positive HNSCC.

4. The composition of claim 2, wherein the HPV-positive cancer is a metastasis.

5. The composition of any one of claim 1 to 4, wherein the Shiga Toxin B subunit (STxB) has the sequence SEQ id n°1, and a STxB fragment refers to a polypeptide having a length of at least 50 amino acids, said fragment having preferably the sequence SEQ id n°2.

6. The composition of any claim 1 to 5, wherein said at least one HPV epitope is a HPV16 epitope.

7. The composition of claim 6, wherein said at least one HPV16 epitope is from the E6 or E7 protein of HPV16, preferably said HPV16 epitope is from HPV16 E7 protein and is selected in the group comprising the peptides consisting of amino acids 5-18, 17-34, 35-50, 43-57, 43-77, 49-57, 50-62, 69-82 of the HPV 16 E7 protein.

8. The composition of claim 7, wherein Preferably, said HPV16 epitope consists in the amino acids 43-57 of the HPV 16 E7 protein (SEQ id n°3; GQAEPDRAHYNIVTF).

9. The composition of any claim 1 to 8, wherein i) the Shiga Toxin B subunit (STxB), fragment or derivative thereof; and ii) the at least one cancer associated epitope are covalently linked using bifunctional protein coupling agents or in a fusion protein.

10. The composition of claim 9, wherein i) the Shiga Toxin B subunit (STxB), fragment or derivative thereof; and ii) the at least one cancer associated epitope are covalently linked using a bifunctional protein coupling agents.

11. The composition of claim 10, wherein i) the Shiga Toxin B subunit (STxB) derivatives refer to the SEQ id n°4 and SEQ id n°5, preferably to SEQ id n°5.

12. The composition of any claim 1 to 11, wherein i) the Shiga Toxin B subunit (STxB), fragment or derivative thereof; and ii) the at least one cancer associated epitope are covalently linked in a fusion protein.

13. The composition of any claim 1 to 12, wherein the radiotherapy can be applied at a dose in a range from about 10 to 55 Gy, preferably from about 15 to 50 Gy, such as 20 to 40Gy, concretely from about 20 to 35 Gy, and more concretely from about 25 to 30 Gy.

14. The composition of any claim 1 to 13, wherein the composition and the radiotherapy are administrated currently, separately, or sequentially.

15. The composition of any claim 1 to 14, wherein the composition is administrated before the irradiation.

16. The composition of any claim 1 to 15, wherein the composition is for use in the prevention or treatment of a subject suffering from a cancer by a parenteral administration.
